# FASCICULE DE BREVET EUROPEEN

(11) **EP 0 709 075 B1**
(45) Date de publication et mention de la délivrance du brevet: **29.08.2001**
(21) Numéro de dépôt: 95420301.4
(22) Date de dépôt: 27.10.1995
(51) Int. Cl.: A61F 2/38

(54) **Implant fémoral pour prothèse unicompartimentale du genou**
Femorales Implantat für eine einseitige Knieprothese
Femoral implant for an unicompartimental knee prosthesis

(30) Priorité: 27.10.1994 FR 9413093
(43) Date de publication de la demande: 01.05.1996
(73) Titulaire: Merck Biomaterial France, 26000 Valence (FR); Bascoulergue, Gérard, F-62520 Le Touquet (FR); Charret, Philippe, 69270 Fontaine sur Saone (FR); Fayard, Jean-Philippe, F-42170 St. Just St. Rambert (FR); Hulin, Paul Henri, 89000 Auxerre (FR); Peyrot, Jacques, 69160 Tassin la Demi Lune (FR)
(72) Inventeur: Bascoulergue, Gérard, F-62520 Le Touquet (FR); Charret, Philippe, F-69270 Fontaine sur Saone (FR); Dupre-Latour, Laurent, F-42580 L'Etrat (FR); Fayard, Jean-Philippe, F-42170 St. Just St. Rambert (FR); Hulin, Paul Henri, F-89000 Auxerre (FR); Peyrot, Jacques, F-69160 Tassin la Demi Lune (FR); Collomb, Jean, F-26800 Portes les Valence (FR)
(74) Mandataire: Ropital-Bonvarlet, Claude

(56) Documents cités:
- EP-A- 0 328 463
- FR-A- 2 677 880
- US-A- 4 719 908

## Description

La présente invention concerne le domaine des prothèses osseuses et elle vise, plus particulièrement, celui des prothèses du genou et, plus spécifiquement encore, le domaine des prothèses unicompartimentales du genou.

Dans le domaine technique ci-dessus, il est connu depuis longtemps déjà de proposer des systèmes articulaires artificiels, visant à remplacer l'articulation naturelle constituée par la conformation épiphysaire basse du fémur, par la conformation épiphysaire complémentaire haute du tibia et par l'élément fémoropatellaire.

Toutes les propositions connues jusqu'à présent, qu'elles soient implantées couramment ou non, s'appuient sur une constitution d'un tel système articulaire artificiel, à partir de deux éléments principaux destinés à être respectivement adaptés, après résection osseuse, sur la conformation épiphysaire basse du fémur et sur la conformation épiphysaire complémentaire, haute du tibia.

L'invention concerne, plus spécifiquement, les propositions relatives aux éléments d'articulation destinés à être implantés sur la conformation épiphysaire basse du fémur.

Le domaine spécifique de l'invention concerne l'adaptation prothétique unicompartimentale, par opposition aux prothèses pluricompartimentales du genou, c'est-à-dire celles visant à substituer à l'articulation naturelle une prothèse complète prenant en compte les conformations congruentes, bi-compartimentales ou tri-compartimentales des conformations épiphysaires basse du fémur et haute du tibia.

Dans certaines applications, il est possible de recourir à l'implantation d'une prothèse mono-compartimentale ou unicompartimentale intéressant l'un des condyles fémoraux en relation avec la glène complémentaire haute du tibia. Dans ce genre d'application et d'adaptation, la prothèse unicompartimentale comprend deux éléments prothétiques complémentaires et adaptés, spécifiquement prévus pour être implantés respectivement, après résection osseuse, sur l'épiphyse basse du fémur et sur l'épiphyse haute du tibia, en n'intéressant que l'un des compartiments congruents de l'articulation naturelle.

La demande de brevet FR 2 677 880 décrit une prothèse unicompartimentale de ce type, destinée à remplacer le condyle interne du fémur et celui correspondant du tibia. Cette prothèse est constituée, d'une part, d'un implant tibial formé d'une embase recevant un patin de glissement et, d'autre part, d'un implant fémoral monobloc.

Toutefois, une telle prothèse n'apparaît pas de nature à fournir une réponse au problème général posé, en raison du caractère spécifique interne qui lui est attaché. En effet, le problème général de l'implantation d'une prothèse unicompartimentale implique, bien évidemment, de devoir considérer une possible implantation en relation avec le compartiment congruent interne gauche ou externe gauche d'un genou gauche ou interne droit et externe droit d'un genou droit.

L'invention vise une proposition nouvelle dont les éléments structurels sont spécifiquement choisis pour permettre une adaptation dans l'un quelconque des quatre cas de figures qui peuvent être concernés par une prothèse unicompartimentale de genou, susceptible donc d'intéresser le compartiment interne gauche, externe gauche, interne droit ou externe droit.

L'invention vise uniquement l'implant prothétique destiné à être adapté sur l'épiphyse basse du fémur, étant entendu que la contrepartie complémentaire adaptée sur l'épiphyse haute du tibia doit être considérée, bien que complémentaire, comme indépendante des caractéristiques techniques qui sont mises en oeuvre pour permettre une adaptation convenable de l'implant fémoral.

Selon la demande FR 2 677 880, l'implant fémoral, destiné à remplacer un condyle interne défectueux, possède une forme générale d'équerre, définie par une petite branche sensiblement rectiligne et par une grande branche galbée. Ces deux branches sont délimitées par des faces arrière planes et par une face extérieure commune continue présentant le profil et le rayon de courbure d'un condyle interne naturel. Les bords latéraux de la grande branche présentent, enfin, un rayon de courbure correspondant à l'obliquité du condyle osseux interne.

Cependant, une telle définition ne permet pas d'obtenir un implant fémoral aux formes reproductibles et donc d'offrir une prothèse ayant un bon comportement dans tous les cas de figures.

L'objet de l'invention est de proposer des moyens de réalisation qui permettent d'obtenir un implant fémoral pour prothèse unicompartimentale de genou, particulièrement adapté à la fonction d'appui, de flexion-extension et de rotation partielle du genou, en respect des caractéristiques anatomiques de l'articulation et en action combinée ou complémentaire avec les conformations articulaires naturelles, parallèles, subsistantes.

Pour atteindre les objectifs ci-dessus, l'implant fémoral est réalisé sous la forme d'un élément prothétique monobloc, de forme générale en équerre définie par une petite branche sensiblement rectiligne, dite de condyle postérieur, et par une grande branche, dite de condyle distal. Ces branches sont délimitées, d'une part, par des faces arrière d'appui planes et comportant, dans l'angle rentrant qu'elles définissent, des moyens d'implantation osseuse et, d'autre part, par une face extérieure commune continue et courbe à la fois dans un plan longitudinal de l'élément choisi perpendiculaire et médian à la face arrière de la petite branche et transversalement à ce plan.

Selon l'invention, l'implant est caractérisé en ce qu'il comporte :
- une grande branche galbée, de telle sorte que la ligne médiane fictive suivant l'apex de sa courbure transversale présente une première partie prolongeant la ligne médiane fictive de la petite branche, en étant aussi contenue dans le plan longitudinal et au moins une seconde partie s'écartant dudit plan longitudinal en direction du plan sagittal anatomique appartenant à l'articulation fémoro-tibiale à laquelle l'implant est destiné.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est une vue schématique d'une articulation d'un genou en flexion considérée parallèlement au plan sagittal.

La **fig. 2** est une vue schématique frontale de l'articulation du genou selon la ligne **II-II** de la **fig. 1,** étant retenu que cette figure correspond à un genou gauche.

La **fig. 3** est une vue comparable à la **fig. 2,** mais intéressant dans les mêmes dispositions un genou droit.

La **fig. 4** est une perspective modélisée de l'implant fémoral pour prothèse unicompartimentale correspondant à l'invention.

La **fig. 5** est une élévation latérale de l'implant selon la **fig. 4.**

La **fig. 6** est une élévation latérale d'une autre forme de réalisation de l'implant fémoral selon la **fig. 4** et correspondant à un implant de taille 3, représenté à l'échelle 1.

La **fig. 7** est une vue transversale, à l'échelle 1, prise selon la ligne **VII-VII** de la **fig. 6.**

La **fig. 8** est une reconstitution linéaire en coupes transversales successives, de différents plans transversaux, à l'échelle 1, pris selon des plans parallèles identifiés de la **fig. 7.**

Les **fig. 9a, 9b** et **10a** et **10b** sont des vues schématiques mettant en évidence, en comparaison avec les **fig. 2** et **3,** des caractéristiques spécifiques constructives de l'implant conforme à l'invention selon sa destination.

Les **fig. 11, 12** et **13** sont des vues parallèles aux **fig. 6, 7** et **8,** mais correspondant à un implant fémoral externe gauche de taille 3, représenté à l'échelle 1 et avec la **fig. 12** prise selon la ligne **XII-XII** de la **fig. 11.**

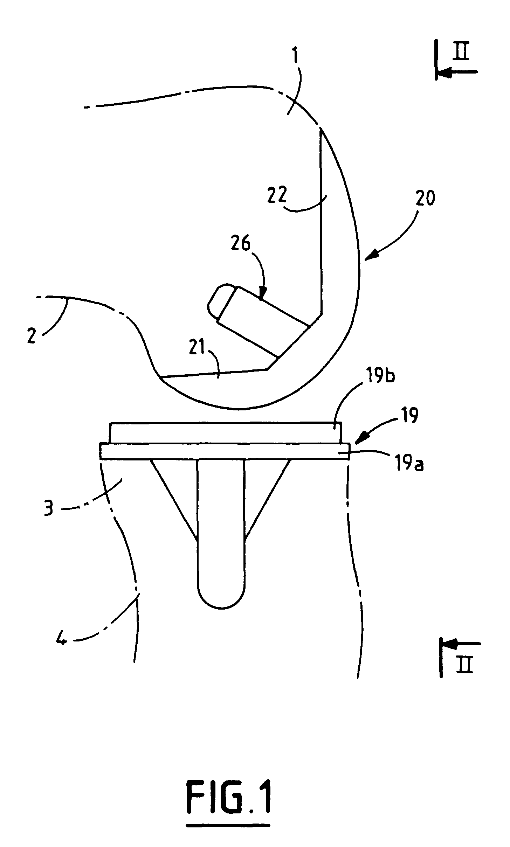

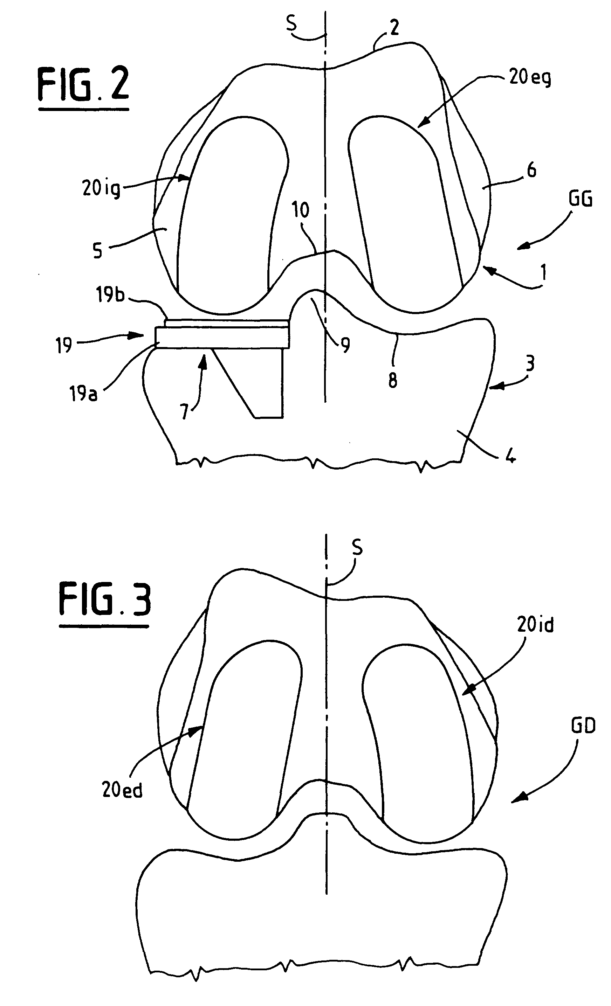

Les **fig. 1** et **2** montrent, de façon schématique, une articulation de genou gauche fléchie, limitée aux conformations épiphysaires congruentes, respectivement basse 1 d'un fémur **2** et haute **3** d'un tibia **4.**

Pour mémoire, la conformation anatomique des épiphyses **1** fait intervenir des massifs condyliens, interne gauche **5** et externe gauche **6,** destinés à coopérer avec les surfaces complémentaires offertes par le plateau tibial **3** qui présente, à cet effet, deux glènes **7** et **8** séparées par un massif épineux **9** complémentaire à une échancrure **10** intercondylienne du fémur.

Une prothèse unicompartimentale consiste à substituer, à l'un des couples congruents **5, 7** ou **6, 8** d'un genou gauche **GG** (fig. **2**) ou d'un genou droit **GD** (fig. **3**), un système artificiel comprenant un implant tibial **19** et un implant fémoral **20.**

Il doit être considéré que l'implant tibial **19** est de type connu, en étant constitué par une embase **19a** implantée, de toute façon convenable, et apte à retenir un patin **19b** réalisé, par exemple, en polyéthylène ou une matière analogue.

L'invention concerne uniquement l'implant fémoral **20** et les caractéristiques constructives, objets de l'invention, valent, pour l'essentiel, à la fois pour un implant fémoral qualifié d'interne gauche **20ig** ou, sous réserve de modifications adaptatives, pour un implant fémoral externe gauche **20eg,** étant entendu qu'au plan anatomique et selon l'invention, comme cela est illustré par les **fig. 2** et **3,** des dispositions symétriques par rapport à un plan sagittal **S,** correspondant à l'articulation à laquelle l'implant est destiné, sont reprises d'un implant fémoral interne gauche **20ig** à un implant fémoral interne droit **20id,** et réciproquement, et d'un implant fémoral externe gauche **20eg** à un implant fémoral externe droit **20ed,** et réciproquement.

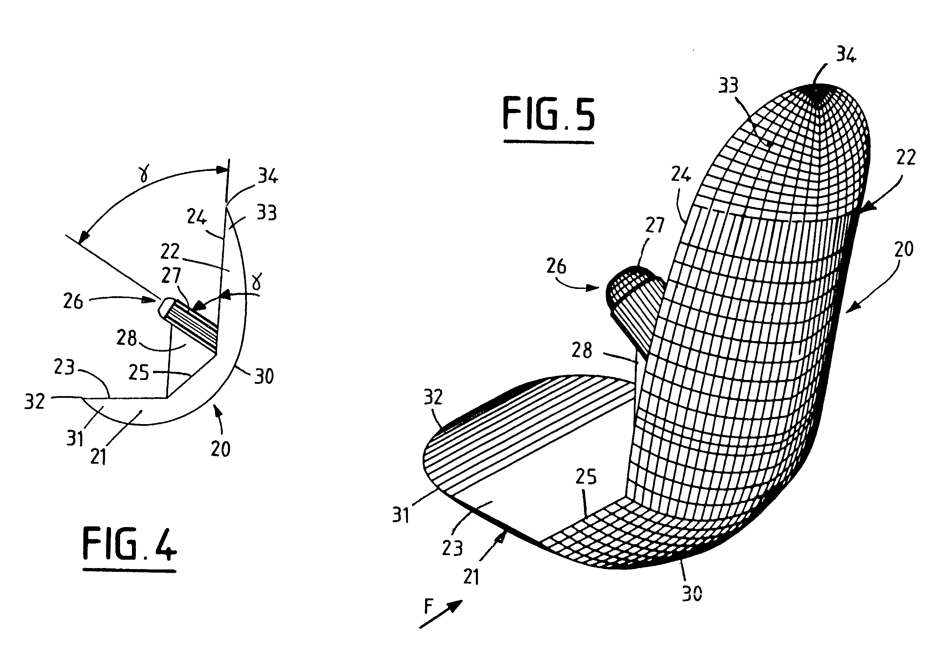

L'implant fémoral conforme à l'invention, destiné à la constitution d'un système articulaire artificiel unicompartimental du genou, est réalisé, comme cela apparaît aux **fig. 1** et **4,** sous la forme d'un élément prothétique monobloc, de forme générale en équerre, définie par une petite branche **21,** dite de condyle postérieur, et par une grande branche **22,** dite de condyle distal. Les définitions de branche de condyle postérieur **21** et de branche de condyle distal **22** sont à considérer en fonction du remplacement prothétique que ces branches assurent par rapport aux conformations naturelles du condyle fémoral, tel que cela ressort de la **fig. 1** montrant, de façon schématique et à titre d'exemple, l'implantation d'un tel élément prothétique.

L'élément prothétique, plus particulièrement mis en évidence par la comparaison des **fig. 4** et **5** est, par ailleurs, réalisé de manière que les branches **21** et **22** soient délimitées par des faces arrière planes **23** et **24,** dites d'appui, et qui, considérées en élévation selon la flèche **F** de la **fig. 4,** délimitent entre elles un angle proche, mais supérieur à un droit, par exemple de 94°. Les faces d'appui **23** et **24** peuvent être lisses ou comporter des accidents de surface, éventuellement associés à un traitement de surface approprié pour favoriser la liaison intime avec la matière osseuse en vis-à-vis de laquelle ces faces sont placées après résection des massifs correspondants, comme cela se pratique habituellement.

Les surfaces d'appui **23** et **24** comportent, dans l'angle rentrant qu'elles définissent, de préférence mais non exclusivement, un pan incliné intermédiaire **25** qui vient réduire la profondeur de l'angle rentrant délimité par les faces **23** et **24** auxquelles ce pan incliné se raccorde.

Les faces **23** et **24** comportent, par ailleurs, dans l'angle rentrant qu'elles délimitent, un moyen d'implantation osseuse **26** qui peut être constitué de plusieurs façons différentes. Dans une première forme de réalisation selon la **fig. 5,** le moyen **26** comprend un téton **27** s'étendant selon une orientation ou un angle γ qui est différent de 45° par rapport à la face **24.** Le téton **27** est raccordé au pan incliné **25** par un gousset **28** pouvant, le cas échéant, joindre également la surface **23.** Le téton **27** est, de préférence, du type cylindrique, cannelé axialement, mais des conformations extérieures différentes peuvent être retenues.

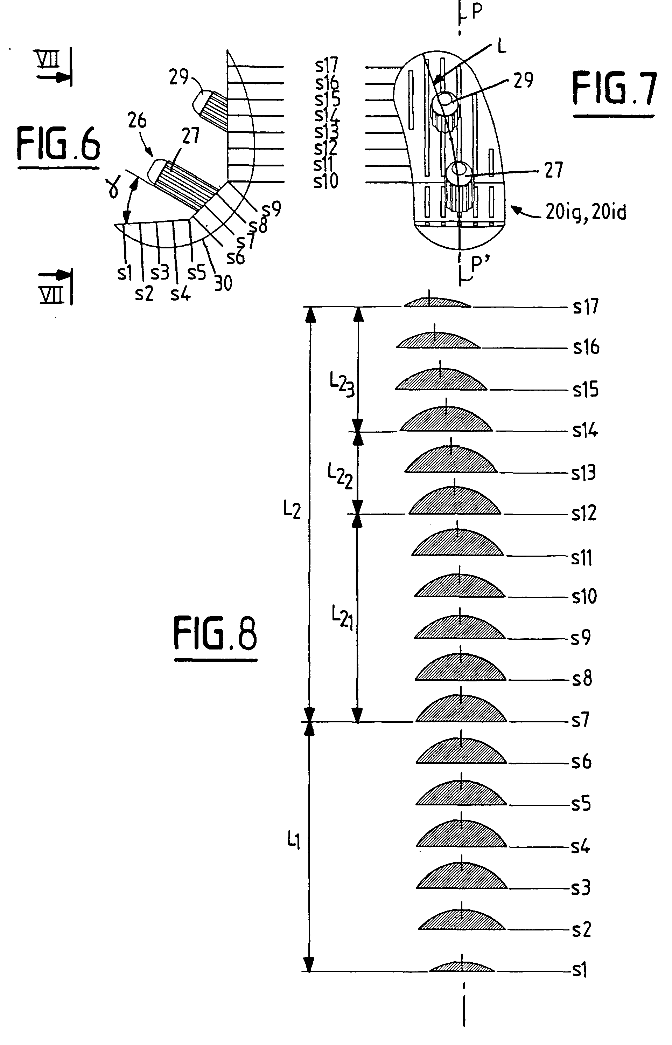

Dans la forme de réalisation illustrée par la **fig. 6,** les moyens d'implantation osseuse **26** comprennent, par ailleurs, un second téton **29** s'étendant de façon parallèle au téton **27,** à partir de la partie sensiblement médiane de la surface arrière **24.** L'angle γ, concernant le téton **27,** est voisin de 30° et, de préférence, compris entre 26° et 30°.

De cette manière, le téton **27** se trouve dans une direction sensiblement parallèle aux contraintes principales exercées par l'articulation sur l'implant, lorsque le genou est dans une position de fléchissement moyen. Or, cette position correspond à la position la plus fréquente du genou, de sorte que l'orientation retenue évite une sollicitation excessive du téton **27.**

L'implant fémoral est, par ailleurs, réalisé de manière que les branches **21** et **22** soient délimitées par une face extérieure commune **30** continue et courbe, à la fois dans le plan longitudinal **P-P'** de l'élément prothétique, tel qu'il est matérialisé à la **fig. 7** et transversalement à ce plan, tel que cela est matérialisé par la **fig. 8** décrite plus en détail dans ce qui suit.

L'implant fémoral selon l'invention est réalisé de manière à comporter des caractéristiques constructives qui sont communes, en respectant toutefois la condition de conformation symétrique par rapport au plan sagittal anatomique **S,** pour un implant interne gauche et un implant interne droit, d'une part, et pour un implant fémoral externe gauche ou externe droit, d'autre part.

Les caractéristiques constructives communes font intervenir une conformation de la petite branche **21,** telle qu'elle présente une partie terminale **31** s'amincissant en direction d'un bord **32** de forme courbe. La petite branche est également définie par une ligne médiane fictive **L1** suivant l'apex de la courbure transversale se développant sur toute la longueur de la branche **21,** cette ligne fictive **L1** étant sensiblement rectiligne et parallèle au plan longitudinal **P-P'** dans lequel elle est contenue, tel que cela ressort des **fig. 7, 8** et **9a,** le plan **P-P'** étant considéré comme médian et perpendiculaire à la face interne **23.**

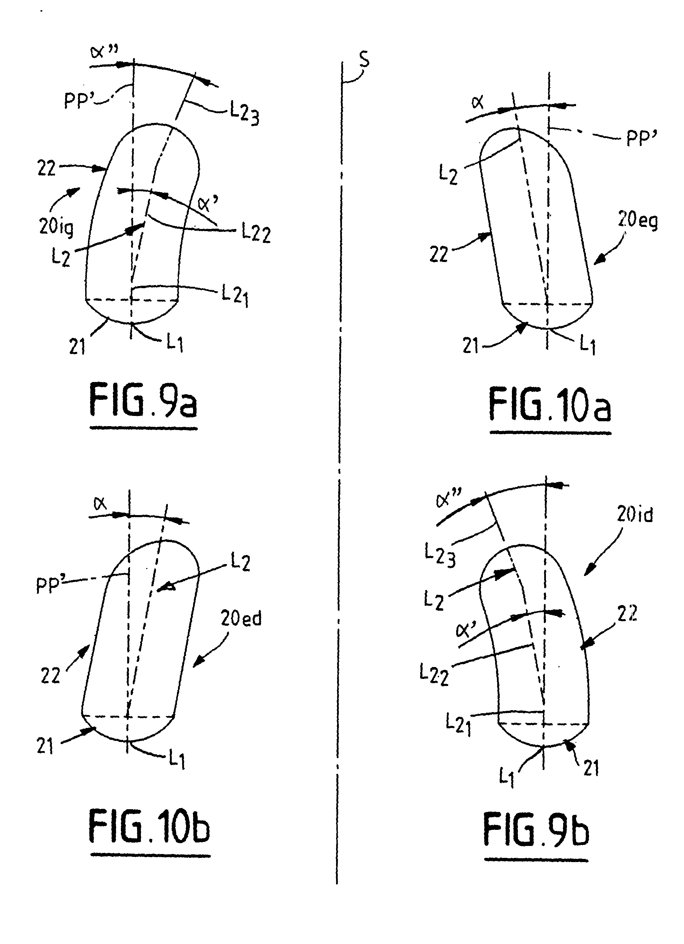

L'implant fémoral, correspondant à un élément prothétique interne gauche, est, par ailleurs, réalisé selon l'invention de telle manière que la grande branche **22** se trouve galbée, de telle sorte qu'elle comporte ou qu'elle soit définie par une ligne médiane fictive **L2** s'établissant dans le prolongement de la ligne fictive **L1** et suivant l'apex de la courbure transversale évolutive que présente la grande branche dont la partie terminale **33** s'amincit pour être définie par un bord extrême **34** courbe. La conformation de la grande branche **22** est choisie de manière que la ligne fictive médiane **L2** présente une première partie **L2**_{**1**}**,** prolongeant la ligne médiane fictive **L1,** en étant elle aussi rectiligne et contenue dans le plan longitudinal **P-P'.** La ligne médiane fictive **L2** comporte ensuite une seconde partie **L2**_{**2**}**,** s'écartant du plan longitudinal **P-P'** en direction du plan anatomique sagittal **S** en faisant, avec le plan longitudinal **P-P',** un angle α' compris entre 10 et 12°, dans le cas d'un implant interne gauche, une valeur de 11,25° étant généralement retenue, comme cela apparaît plus clairement dans la vue schématique de face selon la **fig. 9a.**

Dans le cas de réalisation d'un implant fémoral interne droit, la même valeur que ci-dessus est retenue, en considération d'une réalisation symétrique par rapport au plan sagittal **S.** Ainsi, en considérant les **fig. 9a** et **9b,** par analogie avec les **fig. 2** et **3,** on constate que l'angle α" est de même valeur, mais de sens inversé, pour un implant fémoral interne gauche et pour un implant fémoral interne droit.

Dans le cas de réalisation d'un implant interne gauche ou interne droit, la ligne fictive **L2** comprend une troisième partie **L2**_{**3**}**,** prolongeant la partie **L2**_{**2**}**,** en présentant un angle α" de plus grande valeur angulaire par rapport au plan longitudinal **P-P'.** L'angle α" est, par exemple, compris entre 21 et 23° et une valeur de 22° est généralement retenue.

La partie **L2**_{**3**} s'établit à la suite de la partie **L2**_{**2**} et les sections droites transversales correspondantes, qui sont intéressées par cette évolution de conformation, apparaissent plus particulièrement à l'analyse des **fig. 6** à **8.** Sur la base de la **fig. 8,** les différentes sections droites transversales **s** sont échelonnées et considérées comme prises selon les plans correspondants illustrés par la **fig. 6,** plans désignés, à cette fin, par les mêmes références que celles des différentes sections.

Dans le cas de réalisation d'un élément fémoral externe gauche ou externe droit, des dispositions constructives semblables sont réservées pour la petite branche **21** de condyle postérieur. Par contre, pour la grande branche **22,** une conformation spécifique est choisie, de telle manière que la ligne fictive **L2,** suivant l'apex de la courbure transversale, s'écarte en direction du plan sagittal **S** par rapport au plan longitudinal **P-P'** d'une valeur angulaire α constante, comprise entre 9 et 11° et, plus particulièrement, sensiblement égale à 10°.

Les **fig. 10a** et **10b** sont des vues, analogues aux **fig. 9a** et **9b,** et montrent le caractère symétrique par rapport au plan sagittal **S,** des dispositions constructives externes pour un implant externe gauche et pour un implant externe droit.

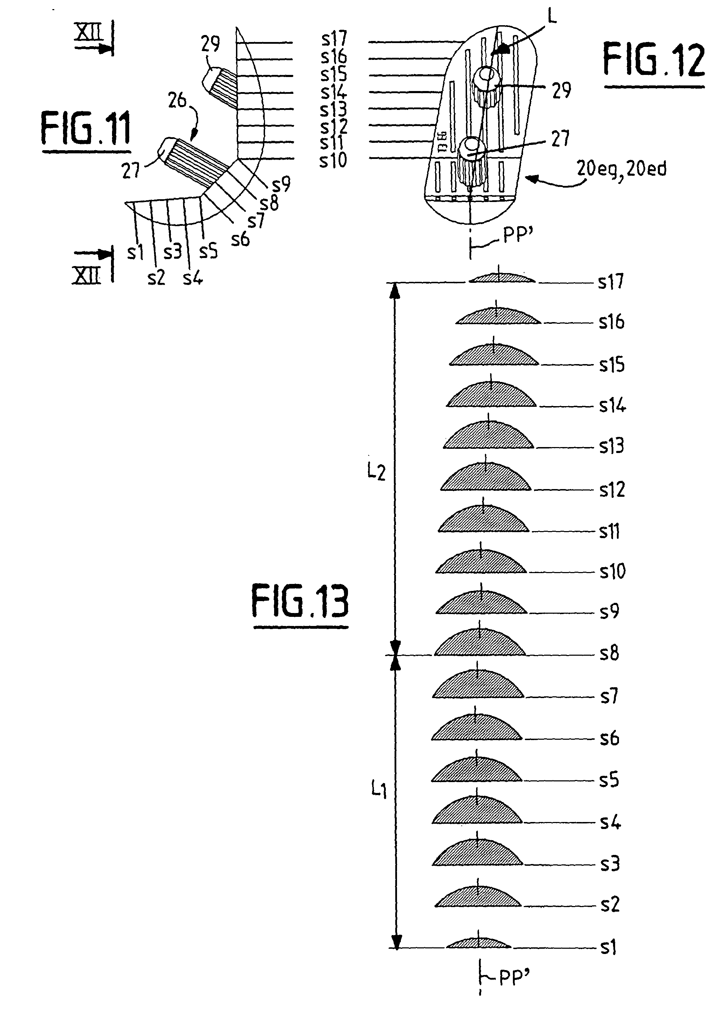

Les **fig. 11** à **13** sont des vues analogues aux **fig. 6** à **8,** montrant les mêmes caractéristiques constructives mises en évidence à partir de la succession de sections transversales s portant les mêmes références.

Ainsi, et selon une autre caractéristique de l'invention, chaque implant fémoral est réalisé de telle manière que chaque branche qu'il comporte, considérée en vue en plan par sa face arrière, présente une conformation oblongue qui est plus particulièrement réniforme pour la grande branche d'un implant fémoral interne gauche ou d'un implant fémoral interne droit.

Les dispositions constructives ci-dessus permettent d'assurer une implantation anatomique adaptée en limitant la résection osseuse nécessaire pour l'adaptation d'une prothèse unicompartimentale, tout en assurant un maintien parfait par l'intermédiaire des moyens d'implantation osseuse **26** dont l'orientation du ou des tétons **27** ou **28** est choisie pour éviter tout glissement intempestif en flexion maximale ou en flexion partielle et pour permettre une résection pratique dans une position de flexion voisine de 90°.

L'implant fémoral décrit ci-dessus est, de préférence, réalisé en métal et, plus particulièrement, en un alliage de chrome-cobalt et possède des surfaces ou des faces arrière comportant des macrostructures pouvant recevoir des revêtements de surface de type bio-actif favorisant la liaison avec le massif osseux réséqué. L'implant fémoral est, par ailleurs, réalisé de manière à présenter, au contraire, une surface extérieure **30** polie par tous moyens appropriés pour favoriser le contact de surface non dégradant avec l'implant tibial **19.**

L'invention n'est pas limitée aux exemples décrits et représentés car diverses modifications peuvent y être apportées sans sortir de son cadre.

## Revendications

1. Implant fémoral pour prothèse unicompartimentale du genou, du type monobloc, de forme générale en équerre définie par :
- une petite branche **(21)** sensiblement rectiligne, dite de condyle postérieur,
- et par une grande branche galbée, dite de condyle distal,
ces branches étant délimitées, d'une part, par des faces arrière d'appui **(23** et **24)** planes et comportant, dans l'angle rentrant qu'elles définissent, des moyens d'implantation osseuse et, d'autre part, par une face extérieure commune **(30),** continue et courbe à la fois, dans un plan longitudinal **(P-P')** de l'élément, choisi perpendiculaire et médian à la face arrière **(23)** de la petite branche et transversalement à ce plan,
**caractérisé en ce que** l'implant comporte :
- une grande branche galbée, de telle sorte que la ligne médiane fictive **(L2)** suivant l'apex de sa courbure transversale présente une première partie **(L2**_{**1**}**)** prolongeant la ligne médiane fictive de la petite branche, en étant aussi contenue dans le plan longitudinal et au moins une seconde partie **(L2**_{**2**}**)** s'écartant dudit plan longitudinal en direction du plan sagittal anatomique **(S)** appartenant à l'articulation fémoro-tibiale à laquelle l'implant est destiné.

2. Implant fémoral selon la revendication 1, **caractérisé en ce qu**'il présente, par rapport au plan sagittal de l'articulation fémoro-tibiale à laquelle il est destiné :
- lorsqu'il est réalisé en tant qu'implant externe gauche, une forme générale symétrique par rapport à un implant externe droit et réciproquement,
- et, lorsqu'il est réalisé en tant qu'implant interne gauche, une forme générale symétrique par rapport à un implant interne droit et réciproquement.

3. Implant fémoral selon la revendication 1 ou 2, **caractérisé en ce que** la ligne fictive médiane **(L2)** de la branche de condyle distal **(22)** s'écarte du plan longitudinal **(P-P')** d'un angle α compris entre 9 et 11°, dans le cas d'un implant externe gauche **(20eg)** ou externe droit **(20ed),** et d'un angle α' compris entre 10 et 12°, dans le cas d'un implant interne gauche **(20ig)** ou interne droit **(20id)**.

4. Implant fémoral selon la revendication 3, **caractérisé en ce que** l'angle α présente une valeur voisine de 10°, tandis que l'angle α' présente une valeur voisine de 11,25°.

5. Implant fémoral selon l'une des revendications 1 à 4, **caractérisé en ce que**, pour un implant interne gauche **(20ig)** ou interne droit **(20id),** la ligne médiane fictive **(L2)** suivant l'apex de la grande branche **(22)** comporte une extrême partie **(L2**_{**3**}**)** présentant un écartement α" de même sens, mais de valeur angulaire supérieure à celle de l'angle **α** '.

6. Implant fémoral selon la revendication 5, **caractérisé en ce que** l'angle α" est compris entre 21 et 23°.

7. Implant fémoral selon la revendication 1, **caractérisé en ce que** les deux branches **(21, 22)** possèdent des parties terminales **(31, 33)** amincies qui sont définies chacune par un bord transversal courbe **(32** ou **34).**

8. Implant fémoral selon la revendication 1 ou 7, **caractérisé en ce que** la petite branche **(21)** et la grande branche **(22)** présentent chacune, en vue en plan perpendiculairement à la face arrière d'appui, une forme oblongue.

9. Implant fémoral selon la revendication 8, **caractérisé en ce que** la forme oblongue de la branche de condyle distal **(22)** d'un élément interne gauche ou interne droit est réniforme.

10. Implant fémoral selon la revendication 1, **caractérisé en ce que** les moyens d'implantation osseuse **(26)** comprennent, au moins, un téton **(27)** s'élevant à partir de l'angle rentrant défini par les faces arrière des branches **(21, 22)** qui sont raccordées entre elles par un pan intermédiaire incliné **(25)**, le téton **(27)** faisant un angle γ différent de 45°, par rapport à l'une quelconque des faces arrière **(23** ou **24).**

11. Implant fémoral selon la revendication **10, caractérisé en ce que** l'angle γ est voisin de 30° par rapport à la face arrière **(23)** de la branche de condyle postérieur **(21).**

12. Implant fémoral selon la revendication 10 ou 11, **caractérisé en ce que** le téton **(27)** est formé à partir du pan intermédiaire **(25)** et se trouve relié à la face arrière de la branche de condyle postérieur par un gousset **(28).**

13. Implant fémoral selon l'une des revendications 10 à 12, **caractérisé en ce que** les moyens d'implantation osseuse comprennent un second téton **(29)** s'élevant à partir de la face arrière **(24)** de la branche de condyle distal **(22)** selon une direction parallèle à celle du premier téton **(27).**

## Patentansprüche

1. Femurimplantat für einstückige, unikondyläre Kniegelenkprothesen, allgemein in rechtwinkliger Form, definiert durch einen im wesentlichen geraden, kleinen Arm (21), dem sogenannten posterioren Kondylenarm und einen gewölbten, großen Arm, dem sogenannten distalen Kondylenarm, wobei diese Arme einerseits von ebenen rückwärtigen Auflageflächen (23 und 24), die in dem durch diese Flächen gebildeten einspringenden Winkel die Knochenimplantationsmittel enthalten, und andererseits von einer gleichzeitig durchgehenden und gebogenen gemeinsamen äußeren Fläche (30) in einer Längsebene (P-P') des Elements begrenzt werden, die senkrecht und median zur rückwärtigen Fläche (23) des kleinen Arms und quer zu dieser Ebene gewählt ist,
**dadurch gekennzeichnet**, daß das Implantat einen großen gewölbten Arm umfaßt, in der Art, daß die fiktive Medianlinie (L₂), die dem Apex seiner querverlaufenden Rundung folgt, einen ersten Teil (L2₁), der die fiktive Medianlinie des kleinen Arms verlängert und zugleich in der Längsebene liegt, und mindestens einen zweiten Teil (L2₂) aufweist, der sich von dieser Längsebene weg in Richtung auf die anatomische Sagittalebene (S) entfernt, die dem Tibiofemoralgelenk angehört, für das das Implantat bestimmt ist.

2. Femurimplantat nach Anspruch 1,
**dadurch gekennzeichnet**, daß das Implantat in Bezug zur Sagittalebene des Tibiofemoralgelenks, für das es bestimmt ist, eine allgemein symmetrische Form gegenüber einem rechten äußeren Implantat und umgekehrt aufweist, wenn das Implantat als linkes äußeres Implantat ausgeführt ist, und eine allgemein symmetrische Form gegenüber einem rechten inneren Implantat und umgekehrt aufweist, wenn das Implantat als linkes inneres Implantat ausgeführt ist.

3. Femurimplantat nach Anspruch 1 oder 2,
**dadurch gekennzeichnet**, daß sich die fiktive Medianlinie (L₂) des distalen Kondylenarms (22) bei einem linken äußeren (20eg) oder rechten äußeren (20ed) Implantat mit einem Winkel α von 9 bis 11° von der Längsebene (P-P') entfernt, bei einem linken inneren (20ig) oder rechten inneren (20id) Implantat mit einem Winkel α' von 10 bis 12°.

4. Femurimplantat nach Anspruch 3,
**dadurch gekennzeichnet**, daß der Winkel α einen Wert von annähernd 10° aufweist, wogegen der Winkel α' eine Wert von annähernd 11,25° aufweist.

5. Femurimplantat nach einem der Ansprüche 1 bis 4,
**dadurch gekennzeichnet**, daß die dem Apex des großen Arms (22) folgende fiktive Medianlinie bei einem linken inneren (20ig) oder rechten inneren (20id) Implantat ein äußeres Teil (L2₃) umfaßt, das in gleicher Richtung einen Abstand α'' aufweist, jedoch mit einem Winkelwert, der größer ist als der Winkel α'.

6. Femurimplantat nach Anspruch 5,
**dadurch gekennzeichnet**, daß der Winkel α'' zwischen 21 und 23° liegt

7. Femurimplantat nach Anspruch 1,
**dadurch gekennzeichnet**, daß die zwei Arme (21, 22) sich verjüngende Endteile (31, 33) besitzen, die jeweils durch einen bogenförmigen, querverlaufenden Rand (32 bzw. 34) gebildet werden.

8. Femurimplantat nach Anspruch 1 oder 7,
**dadurch gekennzeichnet**, daß der kleine Arm (21) und der große Arm (22) senkrecht zur rückwärtigen Auflagefläche gesehen beide jeweils eine längliche Form aufweisen.

9. Femurimplantat nach Anspruch 8,
**dadurch gekennzeichnet**, daß die längliche Form des distalen Kondylenarms (22) eines linken inneren oder rechten inneren Elements eine nierenförmige Form hat.

10. Femurimplantat nach Anspruch 1,
**dadurch gekennzeichnet**, daß die Knochenimplantationsmittel (26) wenigstens einen Zapfen (27) umfassen, der sich aus dem einspringenden Winkel erhebt, der durch die rückwärtigen Flächen der Arme (21, 22) gebildet wird, die miteinander durch ein schräges Zwischenstück (25) verbunden sind, wobei der Zapfen einen Winkel γ von ungleich 45° aufweist

11. Femurimplantat nach Anspruch 10,
**dadurch gekennzeichnet**, daß der Winkel γ in Bezug zur rückwärtigen Fläche (23) des posterioren Kondylenarms (21) annähernd 30° beträgt.

12. Femurimplantat nach Anspruch 10 oder 11,
**dadurch gekennzeichnet**, daß der Zapfen (27) von dem Zwischenstück ausgehend geformt und durch ein Eckstück (28) mit der rückwärtigen Fläche des posterioren Kondylenarms verbunden ist.

13. Femurimplantat nach einem der Ansprüche 10 bis 12,
**dadurch gekennzeichnet**, daß die Knochenimplantationsmittel einen zweiten Zapfen (29) aufweisen, der sich von der rückwärtigen Fläche (24) des distalen Kondylenarms (22) ausgehend in einer parallel zu dem ersten Zapfen (27) verlaufenden Richtung erhebt.

## Claims

1. A femoral implant for a unicompartmental knee prosthesis of the one-piece type, the implant being generally in the form of a right-angled bracket defined by:
- a substantially rectilinear short branch (21) referred to as the "posterior condyle"; and
- a curved long branch referred to as the "distal condyle";
these branches being defined firstly by plane rear support faces (23 and 24) including bone implantation means in the reentrant angle they define between them, and secondly by a common outer face (30) which is continuous and curved both in a longitudinal plane (P-P') of the element, which plane is selected to be a mid-plane perpendicular to the rear face (23) of the short branch, and transverse relative to said plane;
the implant being **characterized in that** it comprises:
- a long branch that is curved in such a manner that an imaginary mid-line (L₂) running along the apex of its transverse curvature presents a first portion (L₂₁) extending an imaginary mid-line of the short branch while also being contained in the longitudinal plane, and at least one second portion (L₂₂) going away from said longitudinal plane towards the anatomic sagittal plane (S) of the femoral-tibial joint for which the implant is intended.

2. A femoral implant according to claim 1, **characterized in that**, relative to the sagittal plane of the femoral-tibial joint for which it is intended, it presents:
- when made as a left lateral implant, a shape which is generally symmetrical to a right lateral implant, and vice versa; and
- when made as a left medial implant, a shape which is generally symmetrical to a right medial implant, and vice versa.

3. A femoral implant according to claim 1 or claim 2, **characterized in that** the imaginary mid-line (L₂) of the distal condyle branch (22) extends away from the longitudinal plane (P-P') at an angle a lying in the range 9° to 11°, for a left lateral implant (20eg) or a right lateral implant (20ed), and at an angle a' lying in the range 10° to 12° for a left medial implant (20ig) or a right medial implant (20id).

4. A femoral implant according to claim 3, **characterized in that** the angle a has a value close to 10°, while the angle a' has a value close to 11.25°.

5. A femoral implant according to any one of claims 1 to 4, **characterized in that** for a left medial implant (20ig) or a right medial implant (20id), the imaginary mid-line (L₂) running along the apex of the long branch (22) has an end portion (L₂₃) that deviates by an angle a" in the same direction but of magnitude greater than the angle a'.

6. A femoral implant according to claim 5, **characterized in that** the angle a" lies in the range 21° to 23°.

7. A femoral implant according to claim 1, **characterized in that** both branches (21, 22) possess tapering end portions (31, 33) each defined relative to a curved transverse edge (32 or 34).

8. A femoral implant according to claim 1 or claim 7, **characterized in that** the short branch (21) and the long branch (22) when seen in a plan view perpendicular to the rear support face thereof is oblong in shape.

9. A femoral implant according to claim 8, **characterized in that** the oblong shape of the distal condyle branch (22) of a left medial element or a right medial element is kidney-shaped.

10. A femoral implant according to claim 1, **characterized in that** the bone implantation means (26) comprise at least one stud (27) extending from the reentrant angle defined by the rear faces of the branches (21, 22) which are interconnected by a sloping intermediate facet (25), the stud (27) making an angle g relative to either one of the rear faces (23 or 24) where g is not equal to 45°.

11. A femoral implant according to claim 10, **characterized in that** the angle g is close to 30° relative to the rear face (23) of the posterior condyle branch (21).

12. A femoral implant according to claim 10 or claim 11, **characterized in that** the stud (27) is made from the intermediate facet (25) and is connected to the rear face of the posterior condyle branch by a gusset (28).

13. A femoral implant according to any one of claims 10 to 12, **characterized in that** the bone implantation means comprise a second stud (29) projecting from the rear face (24) of the distal condyle branch (22) in a direction parallel to the direction of the first stud (27).
